# EUROPEAN PATENT APPLICATION

(11) **EP 1 813 275 A1**
(43) Date of publication of application: **01.08.2007**
(21) Application number: 05257891.1
(22) Date of filing: 20.12.2005
(51) Int. Cl.: A61K 31/4439, A61K 9/28, A61K 9/20, A61K 9/50

(54) **Lansoprazole orally disintegrating tablets**

(71) Applicant: Teva Pharmaceutical Industries Ltd, 49131 Petah Tiqva (IL)
(72) Inventor: Silver, David Isaac, Netanya 42490 (IL); Antler, Sivan, Modiin 71700 (IL); Shterman, Nava, Petach Tikva 49214 (IL); Di Capua, Simona, Kfar Saba (IL); Ari-Pardo, Limor, Beit Halevi 42870 (IL)
(74) Representative: Nachshen, Neil Jacob

(57) **Abstract**

The invention provides orally disintegrating tablets that readily disintegrates in the mouth, releasing enteric coated drug sub-tablets.

## Description

### Field of the Invention

The invention is directed to enteric coated drugs, such as Lansoprazole. In particular, the invention is directed to an easily swallowed tablet that readily disintegrate in the mouth releasing enteric coated drug sub-tablets.

### Background of the Invention

Lansoprazole, a substituted Benzimidazole, is an inhibitor of gastric (H⁺ + K⁺)-ATPase. Lansoprazole has been shown to be unstable under acidic conditions, and, thus, preferably has an enteric coating to prevent exposure of the drug to acidic conditions prior to absorption in the digestive system.

Enteric coated products are known. For example, U.S. Patent No. 6,706,285 discloses an enteric coated Lansoprazole, having a core and a film of an enteric coating agent on the surface thereof, where the core contains a complex of the Lansoprazole and an ion-exchange resin. Many enteric coated products are formulated as a single monolithic unit, such as that disclosed in U.S. Patent No. 6,706,285, while others comprise multiple units, where the multiple-unit formulations are formulated to improve migration in the digestive track, and to minimize various absorption issues. U.S. Patent 6,328,994 discloses an orally disintegrable Lansoprazole tablet which comprises fine enteric coated "granules," having an average particle diameter of 400 µm or less. European Patent EP 0 723 437B1 discloses an oral pharmaceutical multiple unit tableted dosage form, comprising individually enteric coating layered units characterized in that the enteric coating layer has a particular thickness, and comprises a plasticizer an amount of 15 to 50 percent by weight of the enteric coating layer polymer. As a result, the compression of the individual units mixed with the tablet excipients into the multiple unit tableted dosage form reportedly does not significantly affect the acid resistance of the individually enteric coating layered units.

Many patients find almost any type of tablet hard to swallow, with or without water. Therefore, a need exists for a tablet comprising a drug, such as Lansoprazole, that is easily swallowed without exposing the drug to acidic conditions before absorption in the digestive track. The present invention provides such a tablet.

### Summary of the Invention

The present invention is directed to orally disintegrating tablets, comprising enteric coated sub-tablets, which comprise a drug, and to methods of preparing such tablets and sub-tablets, where the sub-tablets preferably have an average particle diameter of more than 400 µm, and the enteric coat preferably comprises less than 15 percent plasticizer by weight of the enteric coating layer polymer. Preferably, the drug is one that is unstable under acidic conditions, more preferably, the drug is a Benzimidazole derivative, such as Lansoprazole, Pantoprazole, Rabeprazole, Omeprazole, and Esomeprazole. Most preferably, the drug is Lansoprazole. Preferably, orally disintegrating tablets of the invention dissolve rapidly in a patients mouth, releasing the enteric coated sub-tablets, which can be swallowed without water. As the sub-tablets are enteric coated, the sub-tablets pass through the stomach without exposure of the drug to acidic conditions, avoiding degradation of the drug prior to absorption in the patient's digestive tract. The compression of the enteric coated sub-tablets into the orally disintegrating tablets of the invention does not significantly affect the acid resistance of the individually enteric coated sub-tablets.

The orally disintegrating tablets of the invention comprise more than one enteric coated sub-tablet, mixed with one or more tablet excipients. The sub-tablets comprise an inner core, substantially free of any alkaline stabilizing agent, where each core comprises one or more inert core excipients and an acid sensitive drug in, on, or over the inner core; an inert first coating layer, disposed over the acid sensitive drug; an alkaline stabilizing layer, comprising an alkaline stabilizing agent, disposed over the inner core and the acid sensitive drug; and an enteric coating layer, which is preferably polymeric and plasticized with less than 15 percent plasticizer, disposed over the alkaline stabilizing layer, where the inert first coating layer is substantially free of and inert with regard to both the acid sensitive drug and the alkaline stabilizing layer. The inert first coating layer substantially improves the stability of the acid sensitive drug.

The orally disintegrating tablets of the invention preferably comprise inner cores, having at least two sub-populations, each sub-population having a different size distribution. However, the inner cores can be composed of a single population, having a single size distribution. When placed in a mouth, the orally disintegrating tablets of the invention disintegrate, facilitating swallowing of the orally disintegrating tablet by a patient. The inner core can also be an extrusion, comprising the acid sensitive drug and one or more excipients. Preferably, the acid sensitive drug comprises a Benzimidazole derivative, such as Lansoprazole, Pantoprazole, Rabeprazole, Omeprazole, and Esomeprazole. More preferably, the drug comprises Lansoprazole. The orally disintegrating tablets of the invention preferably comprise no more than about 50 percent by weight sub-tablets.

As discussed above, the acid sensitive drug can be in, on, or over the inner core, such that the sub-tablets of the orally disintegrating tablet can comprise a drug layer disposed over the inner core, where the drug layer comprises the acid sensitive drug, the inert first coating layer, disposed over the drug layer, and the alkaline stabilizing layer disposed over the inert first coating layer. At least one of the drug layer, the inert first coating layer and the alkaline stabilizing layer can comprise at least one of a film forming agent and at least one excipient.

In one embodiment, sub-tablets useful in the invention comprise an inner, inert core, such as sugar spheres, coated with an acid labile drug, preferably a Benzimidazole derivative, such as Lansoprazole, Pantoprazole, Rabeprazole, Omeprazole, and Esomeprazole, and, more preferably, Lansoprazole, an inert first coating layer, substantially free of the alkaline stabilizing agent and acid labile drug, a second coating layer, comprising an alkaline stabilizing agent, and an outer coating, comprising an enteric coating. However, the materials of the inert core and the acid labile drug can be combined to form the sub-tablet core by, e.g., extruding a formulated particle composed of an acid labile drug and excipients, without departing from the scope of the invention. An orally disintegrating tablet of the invention, comprising such co-extruded cores, further comprises an inert first coating layer and an outer enteric coating.

Useful film forming agents include hypromellose, i.e., hydroxypropyl methylcellulose, and useful excipients include talc, more preferably, extra fine talc. The ratio of the weight of the drug relative to the total weight of the film forming agent and excipient is preferably from about 1:2 to about 2:1, and, more preferably, about 1:1. The film forming agent and excipient can be used in any useful relative amount, and are preferably used in about equal amounts by weight.

Preferably, the inert first coating layer comprises a film forming agent and an excipient. More preferably, the film forming agent is hypromellose and/or the excipient is talc, preferably, extra fine talc. The weight ratio of film forming agent to excipient is preferably from about 1:1 to about 1:2, and, more preferably, is about 2:3. The alkaline stabilizing agent of the alkaline stabilizing layer preferably comprises a carbonate, such as calcium or magnesium carbonate or a mixture thereof, and, more preferably, the alkaline stabilizing layer further comprises a film forming agent, such as hypromellose. The weight ratio of the alkaline stabilizing agent to the film forming agent is preferably from about 1:1 to about 2:1, and, more preferably, is from about 1:1 to about 3:2.

Preferably, the enteric coating is polymeric, and comprises at least one of hypromellose phthalate and methacrylic and methacrylate copolymers. The methacrylic and methacrylate copolymers are preferably selected from the group consisting of a methacrylic acid copolymer type B, a methacrylic acid copolymer type C, a methacrylic acid, methylmethacrylate, and methylmethacrylate copolymer, a methacrylate copolymer, and mixtures thereof. Such polymeric materials are available under the trade name EUDRAGIT^{®}. The enteric coating can further comprises a plasticizer and/or excipients. When present, the plasticizer is less than 15 percent of the enteric coat polymer. Useful plasticizer materials include, but are not limited to, triethyl citrate, and useful excipients include, but are not limited to talc, preferably, extra fine talc, and titanium dioxide.

The inner cores can be composed of a single population, having a single size distribution. However, the orally disintegrating tablets preferably comprise at least first and second sub-populations of the inner cores, where the inner cores in the first sub-population have smaller diameters than the inner cores of the second population. The weight ratio of the first and second sub-populations of inner cores is preferably from about 1:1 to about 4:1, where the ratio is the weight of the first sub-population to that of the second sub-population. More preferably, the weight ratio is from about 2:1 to about 3:1. Where the acid sensitive drug is Lansoprazole, the first sub-population preferably has a size distribution of diameters of from about 250 to about 350 *µ*m, and the second sub-population has a size distribution of diameters of from about 400 to about 500 *µ*m. Where an orally disintegrating tablet comprises a single population of sub-tablets, having a single size distribution, the sized distribution is preferably within the range of from about 250 to about 500 *µ*m. When coated, the diameter of the sub-tablets becomes much greater than the diameter of the inner cores from which they are made, and, thus, a sub-population of inner cores with an average diameter of 300 µm will typically become, after coating, sub-tablets with an average diameter in excess of 400 µm, and a sub-population of inner cores with an average diameter of 450 µm will typically become, after coating, sub-tablets with an average diameter in excess of 600 µm.

Preferably, the orally disintegrating tablet is a compressed tablet comprising the sub-tablets and tablet excipients, where at least one of the excipients preferably functions as a disintegrant. The tablet excipients preferably comprise at least one of a starch or cellulose, a hydrated sugar, and silica, where, more preferably, the starch or cellulose is maize starch cellulose powder, the hydrated sugar is lactose monohydrate, and the silica is colloidal silica. The orally disintegrating tablets can further comprise sweeteners and flavorings. Useful sweeteners include, but are not limited to, sugars, aspartame, and other commercially available artificial sweeteners. The orally disintegrating tablets can further comprise a lubricant or plasticizer, such as magnesium stearate.

Preferably, upon exposure of the sub-tablets to a solution having a pH of about 3.5 for about 20 minutes, no more than about 10 percent by weight of the acid sensitive drug is dissolved by the solution, more preferably, no more than about 6 percent by weight of the acid sensitive drug is dissolved by the solution, and, most preferably, no more than about 1 percent by weight of the acid sensitive drug is dissolved by the solution. In a particularly preferred embodiment, upon exposure of the sub-tablets to a solution having a pH of about 3.5 for about 20 minutes, substantially none of the acid sensitive drug is dissolved by the solution, even though the enteric coating on the sub-tablets comprises less than 15 percent, preferably, less than 10 percent plasticizer, by weight of the enteric coating layer polymer.

In one preferred embodiment, orally disintegrating tablets of the invention comprise a plurality of sub-tablets, mixed with one or more excipients, and formed into a tablet shape. The sub-tablets comprise coated inner cores, coated with a coating comprising a drug; an inert first coating layer that is substantially free of any alkaline stabilizing agent and the drug, disposed over the inner core; an alkaline stabilizing layer, comprising an alkaline stabilizing agent, disposed over the inert first coating layer; and an outer enteric coating disposed over the alkaline stabilizing layer. Preferably, the inner core is formed from any useful material that will readily release the drug in the digestive system, is consumable by a patient, and does not degrade the drug. More preferably, the inner core is formed from particles or granules of a sugar, such as sucrose, which can be spherical or any other useful shape. Preferably, the drug is an acid sensitive drug, and, more preferably, is a Benzimidazole derivative, such as Lansoprazole, Pantoprazole, Rabeprazole, Omeprazole, and Esomeprazole. Most preferably, the drug is Lansoprazole.

In a further preferred embodiment, the orally disintegrating tablets comprise a plurality of sub-tablets, mixed with one or more excipients, and formed into a tablet shape. The sub-tablets comprise an inner core, comprising an acid sensitive drug and inert excipients, but free of any alkaline stabilizing agent; an inert first coating layer, substantially free of the drug and alkaline stabilizing agent; an alkaline stabilizing layer, comprising an alkaline stabilizing agent; and an outer coating layer, comprising an enteric coating. Preferably, the inner core is formed from any useful material that will readily release the drug in the digestive system, is consumable by a patient, and does not degrade the drug. More preferably, the inner core is formed from a sugar, such as sucrose. Most preferably, the drug is a Benzimidazole derivative, such as Lansoprazole, Pantoprazole, Rabeprazole, Omeprazole, and Esomeprazole. Most preferably, the drug is Lansoprazole.

The invention is further directed to a method of preparing orally disintegrating tablets. The method comprises obtaining a plurality of inner cores, comprising an excipient and an acid sensitive drug, which is preferably a Benzimidazole derivative, such as Lansoprazole, Pantoprazole, Rabeprazole, Omeprazole, and Esomeprazole, in the core or in a layer over the core, applying an inert first coating layer, substantially free of the acid sensitive drug and alkaline stabilizing agent, over the acid sensitive drug and the inner cores, applying an alkaline stabilizing layer, comprising an alkaline stabilizing agent, over the inert first coating layer, applying an enteric coating layer, which is preferably polymeric, over the alkaline stabilizing layer, thereby forming enteric coated sub-tablets, and then mixing the enteric coated sub-tablets with one or more excipients, forming a tablet mixture, and compressing a portion of the resulting tablet mixture into an orally disintegrating tablet. Preferably, the orally disintegrating tablets comprise inner cores, having at least two sub-populations, the sub-populations having different size distributions, but can be composed of a single population, having a single size distribution.

The method of preparing orally disintegrating tablets of the invention can further comprise applying a layer of the acid sensitive drug over the inner cores, and applying the inert first coating layer over the drug layer, prior to applying the alkaline stabilizing layer.

Preferably, the amount of excipient in the tablet mixture is sufficiently great relative to the amount of enteric coated sub-tablets, and the enteric coating layer is sufficiently flexible, that cracking of the enteric coating during compression of the tablet is minimized, and upon exposure to a solution having a pH of about 3.5 for about 20 minutes, no more than about 10 percent by weight of the acid sensitive drug is dissolved by the solution. Preferably, at least one of the layers is applied by spraying.

In a preferred embodiment, orally disintegrating tablets in accordance with the invention can be prepared by providing inner cores, and preparing separate dispersions of the ingredients of the drug layer, the inert first coating layer, the alkaline stabilizing layer, and the enteric coating, where each dispersion is formed in a solvent. For the dispersions of the drug layer and the inert first coating layer and the alkaline stabilizing layer, the solvent is preferably purified water. For the enteric layer dispersion, the solvent preferably comprises an organic solvent, and, more preferably, is a mixture of acetone and isopropyl alcohol. Most preferably, the isopropyl alcohol and acetone are used in a ratio of about 2:3. The drug layer dispersion is applied over the inner cores, and preferably allowed to dry. The dispersion of the inert first coating layer is applied over the drug layer, and preferably allowed to dry. The dispersion of the alkaline stabilizing layer is applied over the inert first coating layer, and preferably allowed to dry, and the dispersion of the enteric coating is applied over the alkaline stabilizing layer, and preferably allowed to dry. Preferably, each layer is applied by spraying, and, more preferably, using a fluid bed drier. Preferably, the amount of solvent used in each dispersion is sufficient to allow the ready application of the dispersion, while minimizing the drying time.

In a particularly preferred embodiment, the drug layer is deposited on the inner core, and allowed to dry, the inert first coating layer is deposited on the drug layer, and allowed to dry, the alkaline stabilizing layer is deposited on the inert first coating layer, and allowed to dry, and the enteric coating is deposited on the alkaline stabilizing layer, and allowed to dry. More preferably, the each layer is deposited by spraying a dispersion of the material used to form the layer.

The final orally disintegrating tablets are preferably formed by mixing the enteric coated sub-tablets with the other tablet ingredients, and, preferably, pressing the mixture into tablets, comprising the desired dose of the drug.

The invention is further directed to a method of administering an acid sensitive drug, preferably a Benzimidazole derivative, such as, Lansoprazole, Pantoprazole, Rabeprazole, Omeprazole, and Esomeprazole, comprising orally administering at least one orally disintegrating tablet in accordance with the invention to a patient, where the orally disintegrating tablet at least partially disintegrates in the patients mouth, releasing the enteric coated sub-tablets, which are swallowed by the patient with or without the use of water or other fluid.

### Brief Description of the Drawings

Figure 1 illustrates a cross-section of an orally disintegrating tablet of the invention;
Figure 2 illustrates a cross-section of one preferred embodiment of a sub-tablet of an orally disintegrating tablet; and
Figure 3 illustrates a cross-section of a further preferred embodiment of a sub-tablet.

### Detailed Description of the Preferred Embodiments of the Invention

As used herein, the terms "on," "disposed on," and "deposited on" mean that a second material is disposed or deposited directly on, and in physical contact with a first material. The terms "over," disposed over," and "deposited over", as used herein, means that a second material is outside of a first material of the orally disintegrating tablets and sub-tablets of the invention, and can be, but need not be, in contact with the first material. Instead, at least one intervening layer of material can be, but is not necessarily, disposed or deposited over the first material before the second materials is disposed or deposited. The intervening layer of material can be disposed on or disposed over the first material.

As used here in the term "inactive excipient" refers to an excipient that does not reduce the effectiveness of the drug. Also as used herein, the term "enteric" refers to a material that is acid resistant, such that a sub-tablet coated with an enteric material resists dissolution in an acidic environment.

As used herein, the term "sub-tablet" refers to any pellet, granule, powder, minitab, and the like having the sub-tablet structure described herein.

As used herein, the term "inert first coating layer" of a sub-tablet refers to a coating layer that is inert with regard to any drug, core material, and alkaline stabilizing layer or agent in or on a sub-tablet, and is substantially free of any drug or alkaline stabilizing agent.

The present invention is directed to orally disintegrating tablets that are formulated to readily disintegrate in the mouth of a patient, thereby facilitating swallowing by a patient, preferably without requiring water. Preferably, the orally disintegrating tablets of the invention comprise at least one Benzimidazole derivative, such as Lansoprazole, Pantoprazole, Rabeprazole, Omeprazole, and Esomeprazole, but can be formulated for any drug that can be administered orally. Therefore, although the invention is described herein in terms of Lansoprazole orally disintegrating tablets (Lansoprazole ODT), orally disintegrating tablets, comprising other drugs, are within the scope of the invention.

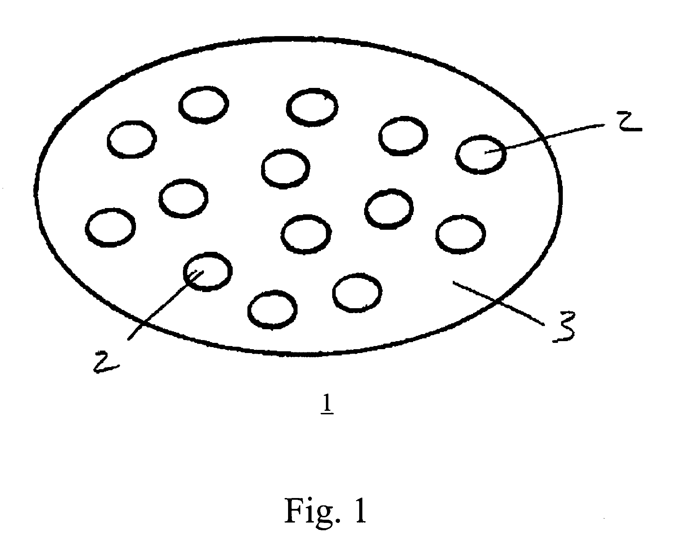

Orally disintegrating tablets in accordance with the invention comprise a plurality of enteric coated sub-tablets containing at least one drug, such as Lansoprazole, mixed with an inactive blend of excipients, and formed into a single tablet, preferably by compression. An orally disintegrating tablet 1 of the invention is illustrated in cross-section in Figure 1. As illustrated in Figure 1, the orally disintegrating tablet 1 of the invention comprises a plurality of enteric coated sub-tablets 2, preferably dispersed within a mixture 3, comprising excipients, flavoring, and sweeteners, and formed into the tablet 1.

Preferably, an enteric coated sub-tablet in an orally disintegrating tablet of the invention comprises:
a) An inner core, such as, but not limited to, sugar particles or granules, which can be spherical or any other useful shape, coated with a drug, preferably, an acid sensitive drug, such as Lansoprazole, where the drug layer preferably comprises a film forming agent, such as hydroxypropyl methylcellulose (hypromellose) and an excipient, such as talc, preferably, extra fine talc, in addition to the drug;
b) A "first coating layer," Subcoat I, which is totally free of both any alkaline stabilizing agent and the drug, where the inert first coating layer preferably comprises a film forming agent, such as hypromellose, and an excipient, such as talc, preferably, extra fine talc;
c) A "alkaline stabilizing layer," Subcoat II, that comprises an alkaline stabilizing, agent, such as magnesium carbonate, and can further comprise a film forming agent, such as hypromellose; and
d) An "outer coating," that comprises an enteric coating, where the enteric coating is preferably polymeric, such as methacrylic and methacrylate copolymers, e.g., those sold under the trade name EUDRAGIT^{®}.

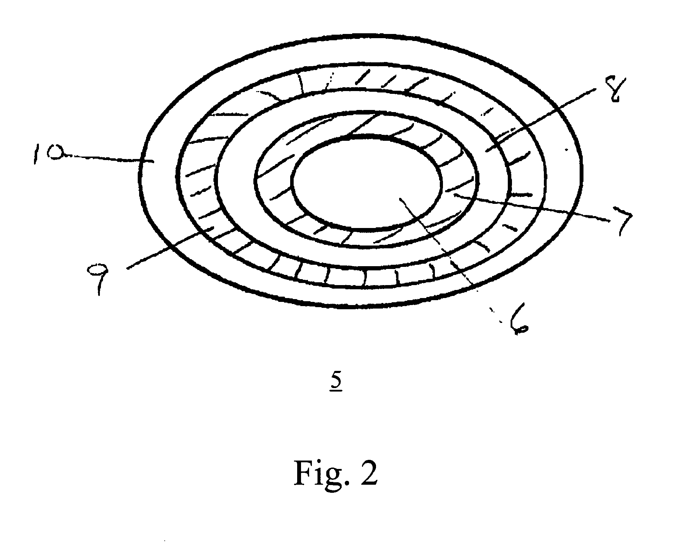

A sub-tablet 5 in accordance with this embodiment of the invention is illustrated in Figure 2. As illustrated in Figure 2, the sub-tablet 5 comprises an inner core 6, a drug layer 7, an inert first coating layer 8, a alkaline stabilizing layer 9, and an enteric coating layer 10.

In a further embodiment, the inner core and the drug are combined into a single unit, produced, e.g., by extruding a formulated particle, comprising the drug, preferably, an acid sensitive drug, such as Lansoprazole, and excipients, without departing from the teachings of the invention. In this embodiment, the sub-tablet of the orally disintegrating tablet comprises: an inner core, comprising the acid sensitive drug and excipients, an inert first coating layer, substantially free of both the drug and any alkaline stabilizing agent, an alkaline stabilizing layer, comprising an alkaline stabilizing agent, and an outer coating agent, comprising an enteric coating.

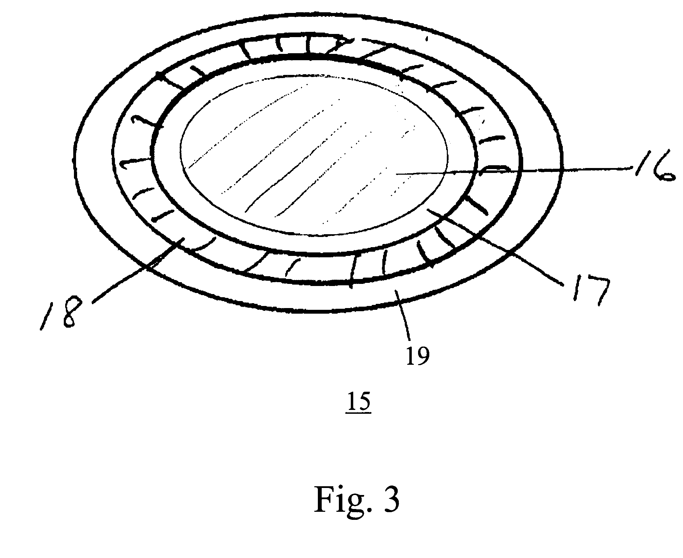

A sub-tablet 15 in accordance with this embodiment of the invention is illustrated in Figure 3. As illustrated in Figure 3, the sub-tablet 15 comprises a core 16, comprising at least one inactive excipient and the acid sensitive drug, an inert first coating layer 17, an alkaline stabilizing layer 18, and an enteric coating layer 19.

The inner cores are preferably sugar spheres, but can have any useful shape, and can comprise any useful material that will readily release the drug in the digestive system, is consumable by a patient, and does not degrade the drug. More preferably, the inner core is formed from a sugar, such as sucrose. Most preferably, the drug is a Benzimidazole derivative, such as Lansoprazole, Pantoprazole, Rabeprazole, Omeprazole, and Esomeprazole. In a preferred embodiment, the drug is Lansoprazole.

Parameters preferably considered during the formulation of orally disintegrating tablets of the invention, such as Lansoprazole ODT, include, but are not necessarily limited to the composition and size distribution of the inner cores, the material used for the enteric coating, which is preferably polymeric, and the excipients surrounding the inner cores. The orally disintegrating tablets of the invention preferably comprise two or more populations, having different size distributions. The size distribution of each population and the relative amounts of the populations are preferably also considered during formulation of the orally disintegrating tablets.

Preferably, the orally disintegrating tablets comprise a mixture of two sub-populations of inner cores, where the weight ratio of the core populations is preferably from about 1:1 to about 4:1, more preferably, from about 2:1 to about 3:1, where the weight ratio is the ratio of the total weight of the smaller cores to the total weight of the larger cores. For Lansoprazole ODT, one sub-population preferably has a range of diameters of from about 250 to about 350 µm, and the second sub-population preferably has a range of diameters of from about 400 to about 500 µm. The use of two sub-populations, having different particle size distributions, provides a more homogeneous mixture of the coated spheres within the external excipients in the final tablet. Table 1 provides examples of different ratios of the various starting particle populations and different weights of cores per final tablet that have been prepared.

The following non-limiting examples are merely illustrative of the preferred embodiments of the present invention, and are not to be construed as limiting the invention, the scope of which is defined by the appended claims.

**Table 1**

| **Materials** | **Example Number** | | | |
|---|---|---|---|---|
| | **1** | **2** | **3** | **4** |
| **Core** | (mg) | (mg) | (mg) | (mg) |
| Sugar spheres 250-350 micron | 90 | 60 | 33.5 | 46.5 |
| Sugar spheres 400-500 micron | 30 | 30 | 16.5 | 23.5 |
| **Total inert part** | **120** | **90** | **50** | **70** |

| **Drug Layer** | | | | |
|---|---|---|---|---|
| Hypromellose | 15 | 15 | 15 | 15 |
| Talc extra fine | 15 | 15 | 15 | 15 |
| Lansoprazole | 30 | 30 | 30 | 30 |
| **Total Drug Layer** | **60** | **60** | **60** | **60** |

| **Subcoat I** | | | | |
|---|---|---|---|---|
| Hypromellose | 12 | 12 | 6 | 6 |
| Talc extra fine | 18 | 18 | 9 | 9 |
| **Total Subcoat I** | **30** | **30** | **15** | **15** |

| **Subcoat II** | | | | |
|---|---|---|---|---|
| Hypromellose | 25 | 16 | 12.5 | 15 |
| Magnesium carbonate | 25 | 24 | 12.5 | 15 |
| **Total Subcoat II** | **50** | **40** | **25** | **30** |

| **Enteric coat** | | | | |
|---|---|---|---|---|
| Methacrylic acid copolymer | 54 | 53 | 62.5 | 88 |
| Talc extra fine | 22 | 21.5 | 25 | 36 |
| Triethylcitrate | 7 | 6.5 | 6 | 9 |
| Titanium dioxide | 3 | 3 | 4 | 7 |
| **Total Enteric coat** | **86** | **84** | **97.5** | **140** |
| **Total coated core** | **346** | **294** | **247.5** | **315** |

| **Tablet** | | | | |
|---|---|---|---|---|
| StarLac (85% Lactose monohydrate & 15% Maize Starch) | 403 | 374 | 420.5 | 400 |
| Strawberry flavor | 10 | 10 | 10 | 11 |
| Aspartame | | 12 | 12 | 13 |
| Colloidal Silicone Dioxide (AEROSIL^{®}) | | 3.5 | 3.5 | 4 |
| Magnesium stearate | 7 | 6.5 | 6.5 | 7 |
| **Total tablet weight** | **766** | **710** | **700** | **750** |

In the examples listed in Table 1, the total weight of inner cores per tablet varied between 50 mg to 120 mg. However, the final tablet weight was fairly similar, due to variation in the other ingredients. The weight ratios of the two populations of cores in Table 1 were in the range of about 2:1 to about 3:1. As will be recognized by those skilled in the art, the total weight of inner cores per tablet and/or the amount of drug per sub-tablet can be adjusted to determine the dosage of tablets containing the sub-tablets.

The tablets exemplified in Table 1 were prepared as follows: An active compound dispersion consisting of Lansoprazole, hypromellose, extra fine talc and purified water was prepared by stirring. First and second sub-coat dispersions were prepared for forming the inert first coating layer and the alkaline stabilizing layer, the first sub-coat dispersion comprising hypromellose, extra fine talc, and purified water, and the second subcoat dispersion comprising hypromellose, magnesium carbonate, and purified water. An outer enteric coating, comprising a dispersion of EUDRAGIT^{®} L-100 55, titanium dioxide, extra fine talc, and triethyl citrate was prepared by stirring with acetone and isopropyl alcohol in a ratio of about 3:2. Sugar spheres were coated by consecutive spraying of the active dispersion subcoats (I + II) and the enteric coat layer using a fluid bed drier equipped with a Würster column (bottom spray).

The polymers used for the enteric coating were also tested. As discussed above, Lansoprazole is unstable in an acidic environment. In order to provide a pharmaceutical composition for oral administration that prevents the drug from degrading in the stomach, the drug is provided with an enteric coating. The dosage form is a tablet that comprises coated particles or granules, preferably in the form of spheres, mixed together with excipients. In order to fully realize the advantages of the formulation, the final tablets are preferably formed on a conventional tablet press without damage to the enteric coating layer of the coated particles during compression. Preferably, to reduce or eliminate the cracking and crushing of the enteric coating layer, a flexible and/or malleable enteric coating layer was provided. To provide such a flexible enteric layer, the tablet formulation preferably contains one or more types of polymer, alone or in a combination.

The flexibility and, more importantly, the robustness and integrity of the enteric layer was tested by comparing the dissolution release profile in acid conditions of the coated spheres versus the dissolution profile of compressed tablets. Formulations using different enteric coated polymers in different amounts and in different combinations are provided in Table 2. Where the enteric coat remains intact, the profile typically remains generally the same.

**Table 2**

| **Materials** | **Example Number** | | | | |
|---|---|---|---|---|---|
| | **10** | **20** | **30** | **40** | **50** |
| **Core** | Values in mg per final Tablet | | | | |
| Sugar spheres 250-350 micron | 46.5 | 46.5 | 46.5 | 46.5 | 46.5 |
| Sugar spheres 400-500 micron | 23.5 | 23.5 | 23.5 | 23.5 | 23.5 |
| **Total inert part** | **70** | **70** | **70** | **70** | **70** |

| **Drug Layer** | | | | | |
|---|---|---|---|---|---|
| Hypromellose | 15 | 15 | 15 | 15 | 15 |
| Talc extra fine | 15 | 15 | 15 | 15 | 15 |
| Lansoprazole | 30 | 30 | 30 | 30 | 30 |
| **Total Drug Layer** | **60** | **60** | **60** | **60** | **60** |

| **Subcoat I** | | | | | |
|---|---|---|---|---|---|
| Hypromellose | 6 | 6 | 6 | 6 | 6 |
| Talc extra fine | 9 | 9 | 9 | 9 | 9 |
| **Total Subcoat I** | **15** | **15** | **15** | **15** | **15** |

| **Subcoat II** | | | | | |
|---|---|---|---|---|---|
| Hypromellose | 15 | 15 | 15 | 15 | 15 |
| Magnesium carbonate | 15 | 15 | 15 | 15 | 15 |
| **Total Subcoat II** | **30** | **30** | **30** | **30** | **30** |

| **Enteric coat** | | | | | |
|---|---|---|---|---|---|
| Methacrylic acid copolymer type C (EUDRAGIT^{®} L-30 D-55) | | 75 | - | - | |
| Methacrylate copolymer (EUDRAGIT^{®} L-100 55) | 78 | | | | 50 |
| Methacrylic acid copolymer type B (EUDRAGIT^{®} S-100) | - | - | - | - | 20 |
| Methacrylic acid, methylmethacrylate, and methylmethacrylate copolymer (EUDRAGIT^{®} FS) | - | 19 | - | - | - |
| Hypromellose phthalate (HPMCP HP-55) | - | - | 56 | 80 | - |
| Polyethylene glycol 6000 | - | - | - | 8 | - |
| Talc extra fine | 31 | 32 | - | - | 26 |
| Triethylcitrate | 8 | 11 | 5.5 | - | 7 |
| Titanium dioxide | 5.5 | 3 | - | | 2 |
| **Total Enteric coat** | **122.5** | **140** | **61.5** | **88** | **105** |
| **Total core** | **297.5** | **315** | **236.5** | **263** | **280** |

| **Tablet** | | | | | |
|---|---|---|---|---|---|
| StarLac (85% Lactose monohydrate & 15% Maize Starch) | 378.5 | 394 | 322 | | |
| CELLACTOSE^{®} 80 (75% lactose monohydrate & 25% cellulose powder) | | | | 234 | 249 |
| Strawberry flavor | 10 | 10.5 | 8 | 7 | 8 |
| Aspartame | 12 | 13 | 10 | 9 | 10 |
| Colloidal Silicone Dioxide (AEROSIL^{®}) | 3.5 | 11 | 9 | 8 | 8 |
| Magnesium stearate | 6.5 | 6.5 | 5 | 5 | 5 |
| **Total tablet weight** | **708** | **750** | **591** | **526** | **560** |

Example 10 exemplifies the use of EUDRAGIT^{®} L-100 55, a methacrylate copolymer, in a solvent, as an enteric coat, and example 20 exemplifies a combination of two polymers, EUDRAGIT^{®} L-30 D-55, a methacrylic acid copolymer type C, and EUDRAGIT^{®} FS, a methacrylic acid, methylmethacrylate, and methylmethacrylate copolymer. In examples 30 and 40, the enteric coat comprises hypromellose phthalate (HPMCP HP-55), and example 50 exemplifies a combination of two polymers, EUDRAGIT^{®} L-100-55 and EUDRAGIT^{®} S-100, methacrylic acid copolymer type B. In each of the examples, the amount of plasticizer was not more than 10 percent by weight.

Dissolution tests were preformed using USP apparatus II at a pH of 3.5 for 20 minutes, at which time the pH of the dissolution medium was adjusted to 5.5 for a further 40 minutes. The results of the dissolution tests are provided in Table 3.

**Table 3**

| | | **Mean Percentage of Drug Dissolution** | | | | |
|---|---|---|---|---|---|---|
| | | **Example Number** | | | | |
| **pH** | Sampling time (Mins.) | **10 L-100¹ 55** | **20 L-30² D-55 & FS** | **30 HPMCP HP-55** | **40 HPMCP HP-55 & PEG** | **50 L-100³ 55 & S-100** |
| **3.5** | 10 | 0.0 | 3.0 | 1.0 | 0.0 | 1.0 |
| | 20 | 0.0 | 5.7 | 6.0 | 1.0 | 3.0 |
| **5.5** | 30 | 6.0 | 19.8 | 66.0 | 20.0 | 16.0 |
| | 40 | 58.0 | 61.6 | 69.0 | 65.0 | 34.0 |
| | 50 | 76.0 | 79.0 | 64.0 | 66.0 | 52.0 |
| | 60 | 74.0 | 72.3 | 52.0 | 60.0 | 56.0 |

| | | | | | | |
|---|---|---|---|---|---|---|
| 1. EUDRAGIT^{®} L-100 55 2. EUDRAGIT^{®} L-30D-55 & FS 3. EUDRAGIT^{®} L-100 55 & S-100 | | | | | | |

The mean percentage drug dissolved after 20 minutes in the acid stage was significantly less than 10 percent by weight in each example. As discussed above, compression during the formation of a final tablet can result in cleavage and crushing of the enteric layer. There appears to be a link between the flexibility of the enteric coating and the dissolution of the sub-tablets in an acidic environment. That is, if the layer is not sufficiently flexible, it can be damaged by the compression forces. As a result, acid can penetrate into the sphere, exposing the active compound to acidic conditions, which degrade the drug.

As illustrated in Table 3, dissolution at a pH of 5.5 was significantly greater than at pH 3.5. The results of example 10 appear to indicate that EUDRAGIT^{®} L-100 55 provides the most flexible and/or least damage prone enteric layer, as no drug was observed to dissolve at a pH of 3.5 after 20 minutes of exposure to those conditions.

The orally disintegrating tablets are intended to disintegrate rapidly in the mouth, and, thus, the selection of the types and amounts of excipients can be crucial to the disintegration of the Lansoprazole ODT in the mouth of a patient. For a tablet to disintegrate rapidly, at least a portion of the excipients must function as a disintegrant. The disintegrant enables the single tablet dosage form, comprising a compressed mixture of excipients and sub-tablets, to disintegrate, and release the sub-tablets.

Preferred excipients include, but are not limited to a spray-dried compound consisting of 85 percent by weight alpha-lactose monohydrate (Ph. Eur./USP-NF) and 15 percent by weight maize starch (Ph. Eur./USP) dry matter, such as STARLAC^{™}, from ROQUETTE, and a spray-dried compound comprising 75 percent by weight alpha-lactose monohydrate (Ph. Eur./USP-NF/JP) and 25 percent by weight cellulose powder (Ph. Eur.) dry matter, such as one CELLACTOSE^{®} 80, from MEGGLE. Examples of useful formulations are provided in Table 4.

**Table 4**

| **Materials** | **Example number** | | | |
|---|---|---|---|---|
| | **100** | **200** | **300** | **400** |
| | (mg) | (mg) | (mg) | (mg) |
| **Enteric coated pellets** | 262.5 | 300.0 | 280.0 | 300.0 |

| **Tablet** | | | | |
|---|---|---|---|---|
| StarLac (85% Lactose monohydrate & 15% Maize Starch) | 335.0 | 408.8 | - | - |
| CELLACTOSE^{®} 80 (75% lactose monohydrate & 25% cellulose powder) | - | - | 350.5 | 267.0 |
| Strawberry flavor | 8.5 | 10.5 | 9.0 | 8.5 |
| Aspartame | 10.5 | 13.0 | 11.0 | 10.0 |
| Colloidal Silicone Dioxide (AEROSIL^{®}) | 3.0 | 11.5 | 10.0 | 9.0 |
| Magnesium stearate | 5.5 | 7.0 | 6.5 | 5.5 |
| Total | 625.0 | 750.8 | 667.0 | 600.0 |

To enable tablet pressing without detectable damage to the enteric coat layer during compression, the ratio of the weight of the coated spheres to that of the excipients in the tablet is very important. Parameters that can affect the optimal ratio of the weight of the enteric coated pellets and that of the whole tablet include, but may not be limited to the uniformity of the content, hardness, friability and disintegration. Results of an evaluation of those parameters are provided in Table 5, where the Lansoprazole ODT were prepared with regard to the following specifications, an average content uniformity of from about 85 to about 115 percent by weight, a relative standard deviation (RSD) of not more than (NMT) 6 percent, a friability of not more than 2 percent, the amount of disintegration, and the hardness.

**Table 5**

| **Parameters** | **Example number** | | | |
|---|---|---|---|---|
| | **100** | **200** | **300** | **400** |
| **% Enteric coated pellets vs. total tablet weight** | **42** | **40** | **42** | **50** |
| **Uniformity of content / RSD** | **106/3.3** | **98.5/3.3** | **111.5/11.9** | **106.2/4.8** |
| **Hardness (SCU) / Friability (%)** | **9/0.8** | **7/4.9** | **9/0.5** | **6/0** |
| **Disintegration (sec)** | **80** | **54** | **67** | **56** |

According to the results provided in Tables 4 and 5, the formulations providing the most desirable physical parameters are Formulation 100, which contains 42 percent by weight enteric coated pellets and STARLAC^{™}, and Formulation 400, which contains 50 percent by weight enteric coated pellets and CELLACTOSE^{®}.

The formulations of the invention are sufficiently stable, such that, upon storage at 30°C and 75 percent relative humidity for 3 months, degradation products are typically formed through acid reaction of the acid sensitive drug in an amount of no more than 0.1 percent.

While it is apparent that the invention disclosed herein is well calculated to fulfill the objects stated above, it will be appreciated that numerous modifications and embodiments may be devised by those skilled in the art. Therefore, it is intended that the appended claims cover all such modifications and embodiments as falling within the true spirit and scope of the present invention.

## Claims

1. An orally disintegrating tablet, comprising more than one enteric coated sub-tablet, mixed with one or more tablet excipients; wherein
the sub-tablets comprise:
an inner core, substantially free of any alkaline stabilizing agent, and comprising one or more inert core excipients;
an acid sensitive drug in, on, or over the inner core;
an inert first coating layer, substantially free of the drug and any alkaline stabilizing agent, disposed over the inner core and the drug;
an alkaline stabilizing layer, comprising an alkaline stabilizing agent, disposed over the inner core and the acid sensitive drug; and
an acid resistant enteric coating layer, disposed over the alkaline stabilizing layer; wherein
the orally disintegrating tablet is formulated to disintegrate when placed in a mouth.

2. The orally disintegrating tablet of claim 1, comprising at least two sub-populations of inner cores, wherein each sub-populations has a different size distribution.

3. The orally disintegrating tablet of claim 1 or 2, wherein the acid sensitive drug comprises a Benzimidazole derivative.

4. The orally disintegrating tablet of claim 1, 2 or 3, wherein the acid sensitive drug comprises Lansoprazole.

5. The orally disintegrating tablet of any preceding claim, wherein the acid sensitive drug is comprised in a drug layer disposed over the inner core.

6. The orally disintegrating tablet of claim 1, further comprising a drug layer, comprising the acid sensitive drug, disposed over the inner core.

7. The orally disintegrating tablet of claim 5 or 6, wherein at least one of the drug layer, the inert first coating layer and the alkaline stabilizing layer comprises at least one of a film forming agent and an excipient.

8. The orally disintegrating tablet of any preceding claim, wherein the alkaline stabilizing agent comprises a carbonate.

9. The orally disintegrating tablet of claim 8, wherein the carbonate is calcium carbonate, magnesium carbonate, or a mixture thereof.

10. The orally disintegrating tablet of any preceding claim, wherein the enteric coating comprises at least one of hypromellose phthalate and methacrylic and methacrylate copolymers.

11. The orally disintegrating tablet of claim 10, wherein the methacrylic and methacrylate copolymers are selected from the group consisting of a methacrylic acid copolymer type B, a methacrylic acid copolymer type C, a methacrylic acid, methylmethacrylate, and methylmethacrylate copolymer, a methacrylate copolymer, and mixtures thereof.

12. The orally disintegrating tablet of any preceding claim, comprising first and second sub-populations of the inner cores, the inner cores in the first sub-population having smaller diameters than the inner cores of the second population, in a weight ratio the first sub-population to the second sub-population of from about 1:1 to about 4:1.

13. The orally disintegrating tablet of claim 12, wherein the weight ratio is from about 2:1 to about 3:1.

14. The orally disintegrating tablet of claim 12 or 13, wherein the acid sensitive drug is Lansoprazole, and the first sub-population has a size distribution of diameters of from about 250 to about 350 µm, and the second sub-population has a size distribution of diameters of from about 400 to about 500 µm.

15. The orally disintegrating tablet of any preceding claim, wherein the inner core is an extrusion, comprising the acid sensitive drug and 1 or more excipients.

16. The orally disintegrating tablet of any preceding claim, wherein the orally disintegrating tablet is a compressed tablet comprising the sub-tablets and excipients.

17. The orally disintegrating tablet of any preceding claim, wherein the tablet excipients comprise at least one of a starch, a cellulose, a hydrated sugar, and silica.

18. The orally disintegrating tablet of claim 17, wherein the starch is maize starch, the cellulose is powdered cellulose, the hydrated sugar is lactose monohydrate, and the silica is colloidal silica.

19. The orally disintegrating tablet of any preceding claim, wherein the enteric coating layer further comprises at least one of a plasticizer and one or more excipients.

20. The orally disintegrating tablet of claim 19, wherein the excipients comprise at least one of titanium dioxide and talc.

21. The orally disintegrating tablet of any preceding claim, wherein upon exposure of the sub-tablets to a solution having a pH of about 3.5 for about 20 minutes, no more than about 10 percent by weight of the acid sensitive drug is dissolved by the solution.

22. The orally disintegrating tablet of any preceding claim, wherein upon exposure of the sub-tablets to a solution having a pH of about 3.5 for about 20 minutes, no more than about 6 percent by weight of the acid sensitive drug is dissolved by the solution.

23. The orally disintegrating tablet of any preceding claim, wherein upon exposure of the sub-tablets to a solution having a pH of about 3.5 for about 20 minutes, no more than about 1 percent by weight of the acid sensitive drug is dissolved by the solution.

24. The orally disintegrating tablet of any preceding claim, wherein upon exposure of the sub-tablets to a solution having a pH of about 3.5 for about 20 minutes, substantially none of the acid sensitive drug is dissolved by the solution.

25. The orally disintegrating tablet of any preceding claim, comprising no more than about 50 percent by weight sub-tablets.

26. A method of preparing enteric coated sub-tablets, comprising:
obtaining a plurality of inner cores, comprising an excipient and an acid sensitive drug in the core or in a layer over the core;
applying an inert first coating layer over the cores and drug;
applying an alkaline stabilizing layer, comprising an alkaline stabilizing agent, over the inert first coating layer; and
applying an enteric coating layer over the alkaline stabilizing layer, forming enteric coated sub-tablets.

27. The method of preparing enteric coated sub-tablets of claim 26, wherein at least one of the layers is applied by spraying.

28. The method of preparing enteric coated sub-tablets of claim 26 or 27, further comprising applying a layer of the acid sensitive drug over the inner cores.

29. The method of preparing enteric coated sub-tablets of claim 26, 27 or 28, wherein the acid sensitive drug is a Benzimidazole derivative.

30. The method of preparing enteric coated sub-tablets of claim 29, wherein the acid sensitive drug is Lansoprazole.

31. A method of preparing orally disintegrating tablets, comprising:
mixing the enteric coated sub-tablets of any of claims 26 to 30 with one or more excipients, forming a tablet mixture; and
compressing a portion of the resulting tablet mixture into orally disintegrating tablets.

32. The method of preparing orally disintegrating tablets of claim 31, wherein the orally disintegrating tablets comprise at least two sub-populations of inner cores, having different size distributions.

33. The method of preparing orally disintegrating tablets of claim 31 or 32, wherein the amount of excipient in the tablet mixture is sufficiently great relative to the amount of enteric coated sub-tablets, and the enteric coating layer is sufficiently flexible, such that cracking of the enteric coating is minimized during compression, and upon exposure to a solution having a pH of about 3.5 for about 20 minutes, no more than about 10 percent by weight of the acid sensitive drug is dissolved by the solution.

34. The method of preparing orally disintegrating tablets of any of claims 31 to 33, wherein the sub-tablets are present in a weight less than that of the excipients.

35. An enteric coated sub-tablet, comprising:
an inner core, substantially free of any alkaline stabilizing agent, and comprising one or more inert core excipients;
an acid sensitive drug in, on, or over the inner core;
an inert first coating layer, substantially free of the drug and any alkaline stabilizing agent, disposed over the inner core and the drug;
an alkaline stabilizing layer, comprising an alkaline stabilizing agent, disposed over the inner core and the acid sensitive drug; and
an acid resistant enteric coating layer, disposed over the alkaline stabilizing layer.

36. An orally disintegrable tablet, comprising:
(i) sub-tablets having an average particle diameter of at least 400 µm, wherein the sub-tablets comprise at least one benzimidazole derivative composition coated by an enteric coating layer, comprising a first component, which is an enteric coating agent, and a second component, which is a plasticizer; and
(ii) an additive; wherein
the tablet has a hardness strength greater than 6 SCU, and is orally disintegrable.

37. The orally disintegrable tablet of claim 36, wherein the plasticizer is present in an amount of less than 15 percent by weight of the enteric coating layer, wherein, upon exposure of the sub-tablets to a solution having a pH of about 3.5 for about 20 minutes, no more than about 10 percent by weight of the acid sensitive drug is dissolved by the solution.

38. The orally disintegrable tablet of claim 36, wherein the plasticizer is present in an amount of 10 percent or less by weight of the enteric coating layer, wherein, upon exposure of the sub-tablets to a solution having a pH of about 3.5 for about 20 minutes, no more than about 10 percent by weight of the acid sensitive drug is dissolved by the solution.

39. The orally disintegrating tablet of claim 36, 37 or 38, wherein the at least one benzimidazole derivative comprises Lansoprazole.

40. Orally disintegrating tablets, comprising more than one enteric coated sub-tablet, mixed with one or more tablet excipients, the sub-tablets comprising at least one benzimidazole derivative; wherein the orally disintegrating tablets comprise first and second sub-populations of inner cores, the inner cores in the first sub-population having smaller diameters than the inner cores of the second population, and wherein the tablets have an average content uniformity of from about 85 to about 115 percent by weight and a relative standard deviation (RSD) of not more than 6 percent.

## Amended claims

### Amended claims in accordance with Rule 86(2) EPC.

**1.** An orally disintegrating tablet, comprising more than one enteric coated sub-tablet, mixed with one or more tablet excipients; wherein
the sub-tablets comprise:
an inner core, substantially free of any alkaline stabilizing agent, and comprising one or more inert core excipients;
an acid sensitive drug in, on, or over the inner core;
an inert first coating layer, substantially free of the drug and any alkaline stabilizing agent, disposed over the inner core and the drug;
an alkaline stabilizing layer, comprising an alkaline stabilizing agent, disposed over the inner core and the acid sensitive drug; and
an acid resistant enteric coating layer, disposed over the alkaline stabilizing layer; wherein
the orally disintegrating tablet is formulated to disintegrate when placed in a mouth.

**2.** The orally disintegrating tablet of claim 1, further comprising at least two sub-populations of inner cores, wherein each sub-populations has a different size distribution.

**3.** The orally disintegrating tablet of claim 1, wherein the acid sensitive drug comprises a Benzimidazole derivative.

**4.** The orally disintegrating tablet of claim 1, wherein the acid sensitive drug comprises Lansoprazole.

**5.** The orally disintegrating tablet of claim 1, further comprising a drug layer disposed over the inner core, the drug layer comprising the acid sensitive drug.

**6.** The orally disintegrating tablet of claim 1, further comprising a drug layer, comprising the acid sensitive drug, disposed over the inner core.

**7.** The orally disintegrating tablet of claim 6, wherein at least one of the drug layer, the inert first coating layer and the alkaline stabilizing layer comprises at least one of a film forming agent and an excipient.

**8.** The orally disintegrating tablet of claim 1, wherein the alkaline stabilizing agent comprises a carbonate.

**9.** The orally disintegrating tablet of claim 8, wherein the carbonate is calcium carbonate, magnesium carbonate, or a mixture thereof.

**10.** The orally disintegrating tablet of claim 1, wherein the enteric coating comprises at least one of hypromellose phthalate and methacrylic and methacrylate copolymers.

**11.** The orally disintegrating tablet of claim 10, wherein the methacrylic and methacrylate copolymers are selected from the group consisting of a methacrylic acid copolymer type B, a methacrylic acid copolymer type C, a methacrylic acid, methylmethacrylate, and methylmethacrylate copolymer, a methacrylate copolymer, and mixtures thereof.

**12.** The orally disintegrating tablet of claim 1, further comprising first and second sub-populations of the inner cores, the inner cores in the first sub-population having smaller diameters than the inner cores of the second population, in a weight ratio the first sub-population to the second sub-population of from about 1:1 to about 4:1.

**13.** The orally disintegrating tablet of claim 12, wherein the weight ratio is from about 2:1 to about 3:1.

**14.** The orally disintegrating tablet of claim 12, wherein the acid sensitive drug is Lansoprazole, and the first sub-population has a size distribution of diameters of from about 250 to about 350 µm, and the second sub-population has a size distribution of diameters of from about 400 to about 500 µm.

**15.** The orally disintegrating tablet of claim 1, wherein the inner core is an extrusion, comprising the acid sensitive drug and 1 or more excipients.

**16.** The orally disintegrating tablet of claim 1, wherein the orally disintegrating tablet is a compressed tablet comprising the sub-tablets and excipients.

**17.** The orally disintegrating tablet of claim 1, wherein the tablet excipients comprise at least one of a starch, a cellulose, a hydrated sugar, and silica.

**18.** The orally disintegrating tablet of claim 17, wherein the starch is maize starch, the cellulose is powdered cellulose, the hydrated sugar is lactose monohydrate, and the silica is colloidal silica.

**19.** The orally disintegrating tablet of claim 1, wherein the enteric coating layer further comprises at least one of a plasticizer and one or more excipients.

**20.** The orally disintegrating tablet of claim 19, wherein the excipients comprise at least one of titanium dioxide and talc.

**21.** The orally disintegrating tablet of claim 1, wherein upon exposure of the sub-tablets to a solution having a pH of about 3.5 for about 20 minutes, no more than about 10 percent by weight of the acid sensitive drug is dissolved by the solution.

**22.** The orally disintegrating tablet of claim 1, wherein upon exposure of the sub-tablets to a solution having a pH of about 3.5 for about 20 minutes, no more than about 6 percent by weight of the acid sensitive drug is dissolved by the solution.

**23.** The orally disintegrating tablet of claim 1, wherein upon exposure of the sub-tablets to a solution having a pH of about 3.5 for about 20 minutes, no more than about 1 percent by weight of the acid sensitive drug is dissolved by the solution.

**24.** The orally disintegrating tablet of claim 1, wherein upon exposure of the sub-tablets to a solution having a pH of about 3.5 for about 20 minutes, substantially none of the acid sensitive drug is dissolved by the solution.

**25.** The orally disintegrating tablet of claim 1, comprising no more than about 50 percent by weight sub-tablets.

**26.** A method of preparing orally disintegrating tablets, comprising:
mixing enteric coated sub-tablets with one or more excipients, forming a tablet mixture; and
compressing a portion of the resulting tablet mixture into orally disintegrating tablets, wherein said enteric coated sub-tablets are prepared by a method, comprising:
obtaining a plurality of inner cores, comprising an excipient and an acid sensitive drug in the core or in a layer over the core;
applying an inert first coating layer over the cores and drug;
applying an alkaline stabilizing layer, comprising an alkaline stabilizing agent, over the inert first coating layer; and
applying an enteric coating layer over the alkaline stabilizing layer, forming enteric coated sub-tablets.

**27.** The method of preparing enteric coated sub-tablets of claim 26, wherein at least one of the layers is applied by spraying.

**28.** The method of preparing enteric coated sub-tablets of claim 26 or 27, further comprising applying a layer of the acid sensitive drug over the inner cores.

**29.** The method of preparing enteric coated sub-tablets of claim 26, 27 or 28 wherein the acid sensitive drug is a Benzimidazole derivative.

**30.** The method of preparing enteric coated sub-tablets of claim 29, wherein the acid sensitive drug is Lansoprazole.

**31.** The method of preparing orally disintegrating tablets of any of claims 26 to 30, wherein the orally disintegrating tablets comprise at least two sub-populations of inner cores, having different size distributions.

**32.** The method of preparing orally disintegrating tablets of any of claims 26 to 31, wherein the amount of excipient in the tablet mixture is sufficiently great relative to the amount of enteric coated sub-tablets, and the enteric coating layer is sufficiently flexible, such that cracking of the enteric coating is minimized during compression, and upon exposure to a solution having a pH of about 3.5 for about 20 minutes, no more than about 10 percent by weight of the acid sensitive drug is dissolved by the solution.

**33.** The method of preparing orally disintegrating tablets of any of claims 26 to 32, wherein the sub-tablets are present in a weight less than that of the excipients.

**34.** An orally disintegrable tablet, comprising:
(i) sub-tablets having an average particle diameter of at least 400 µm, wherein the sub-tablets comprise at least one benzimidazole derivative composition coated by an enteric coating layer, comprising a first component, which is an enteric coating agent, and a second component, which is a plasticizer; and
(ii) an additive; wherein
the tablet has a hardness strength greater than 6 SCU, and is orally disintegrable.

**35.** The orally disintegrable tablet of claim 34, wherein the plasticizer is present in an amount of less than 15 percent by weight of the enteric coating layer, wherein, upon exposure of the sub-tablets to a solution having a pH of about 3.5 for about 20 minutes, no more than about 10 percent by weight of the acid sensitive drug is dissolved by the solution.

**36.** The orally disintegrable tablet of claim 34, wherein the plasticizer is present in an amount of 10 percent or less by weight of the enteric coating layer, wherein, upon exposure of the sub-tablets to a solution having a pH of about 3.5 for about 20 minutes, no more than about 10 percent by weight of the acid sensitive drug is dissolved by the solution.

**37.** The orally disintegrating tablet of claim 34, wherein the at least one benzimidazole derivative comprises Lansoprazole.

**38.** Orally disintegrating tablets, comprising more than one enteric coated sub-tablet, mixed with one or more tablet excipients, the sub-tablets comprising at least one benzimidazole derivative; wherein the orally disintegrating tablets comprise first and second sub-populations of inner cores, the inner cores in the first sub-population having smaller diameters than the inner cores of the second population, and wherein the tablets have an average content uniformity of from about 85 to about 115 percent by weight and a relative standard deviation (RSD) of not more than 6 percent.
